# EUROPEAN PATENT APPLICATION

(11) **EP 1 254 644 A1**
(43) Date of publication of application: **06.11.2002**
(21) Application number: 01303996.1
(22) Date of filing: 01.05.2001
(51) Int. Cl.: A61F 2/06, A61M 25/10

(54) **Variable form stent and deployment arrangement for use therewith**

(71) Applicant: Pan Medical Limited, Glasgow G52 4PX (GB)
(72) Inventor: Nasralla, Max, Gloucestershire, Cheltenham GL52 3NU (GB)
(74) Representative: Bailey, Richard Alan

(57) **Abstract**

A stent implantation arrangement comprises a stent (18) mounted upon a balloon (10), the stent (18) extending over regions (12, 14) of the balloon (10) of different diameters, when the balloon occupies an inflated condition. A stent (18) particularly suitable for use in the implantation and deployment arrangement includes regions of different radial resistance to expansion.

## Description

This invention relates to a medical stent of variable form and to a deployment arrangement for use in deploying and implanting the stent into a patient.

A medical stent is a tubular body designed to be implanted into a blood vessel to support the vessel against collapse or to hold the vessel in a widened condition, for example to avoid restricting blood flow along the vessel. The stent is typically inserted into the vessel whilst occupying a condition in which the stent is of relatively small diameter. Once in the correct location, the stent is expanded to be of a relatively large diameter serving to hold the vessel in a widened condition, or to support the vessel against collapse. The engagement between the stent and the vessel further serves to hold the stent in position.

Stents are available in a large number of designs, for example tubular bodies formed by winding wire to a suitable tubular shape, and stents in the form of a stainless steel tube in which a series of slots are formed by laser cutting, the slots being arranged to allow the expansion of the stent to the enlarged diameter condition. One common technique for expanding the diameter of such a stent is to mount the stent upon a balloon by crimping, position the balloon at the location at which the stent is to be implanted, inflate the balloon to thereby expand the stent, and then apply a vacuum to deflate the balloon to allow removal of the balloon leaving the stent in position.

The balloons used in this technique are typically of generally cylindrical shape, and are used in the implantation of stents which, in their expanded condition, are also of generally cylindrical shape. The balloons are available in a range of diameters. Although in many cases, the use of a stent which, when implanted, is of generally cylindrical shape has the desired effect of holding the vessel within which it is implanted at a desired diameter, there are occasions and anatomical conditions where it may be preferred to implant a stent of a non-cylindrical shape and having regions of two or more different diameters. It is an object of the invention to provide an arrangement whereby a stent can be implanted and whereby the stent, when implanted, is not of generally cylindrical shape and not of uniform diameter. Another object of the invention is to provide a stent suitable for use in such an arrangement.

According to the present invention there is provided a stent deployment and implantation arrangement comprising an inflatable balloon and a stent mounted and crimped upon the balloon, the balloon being of the type which, when inflated, has a useful length which adopts a non-cylindrical shape, wherein the stent is crimped over the useful length of the balloon which, when inflated, takes a non-cylindrical shape.

It will be appreciated that the stent expands during implantation to take the shape of the corresponding part of the balloon and so, in accordance with the invention, a stent can be implanted which is of non-cylindrical shape. By way of example, the balloon may be designed in such a manner that, when inflated, it is of "dog bone" shape having a pair of regions of relatively large diameter separated by a smaller diameter region. The enlarged diameter regions need not be of the same diameter. Further, three or more enlarged diameter regions could be provided, if desired. Alternatively, the balloon could be shaped to include a single large diameter region and a single smaller diameter region. Obviously, a number of other alternatives are possible.

Although the invention may be used with a wide range of stent designs, in a particularly advantageous arrangement the stent is designed to include regions having different radial resistances to expansion.

By arranging the stent in such a manner that it has a relatively high resistance to expansion in those areas where, in its expanded state, the stent is to be of relatively small diameter and a lower resistance to expansion in those areas where the stent is to be of a larger diameter, it will be appreciated that the stent and the balloon work together in ensuring that the stent, when implanted, takes the desired shape.

The variations in the radial resistance to expansion can be achieved, in a stent of the type comprising a plurality of interconnected corrugated rings, each ring being composed of interconnected struts, by arranging for the struts in one part of the stent to have a different strut thickness to those in another part of the stent.

The invention further relates to a stent of the type described hereinbefore.

The invention will further be described, by way of example, with reference to the accompanying drawings, in which:
Figure 1 is a view of a stent crimped upon a balloon prior to inflation of the balloon;
Figure 2 is a view similar to Figure 1 illustrating the arrangement after inflation of the balloon;
Figure 3 is a view of a stent particularly suitable for use in the stent implantation arrangement;
Figure 4 is a view similar to Figure 3 of an alternative stent with different strut thickness; and
Figures 5 to 7 are diagrams illustrating alternate balloons, in their inflated conditions, suitable for use in the invention.

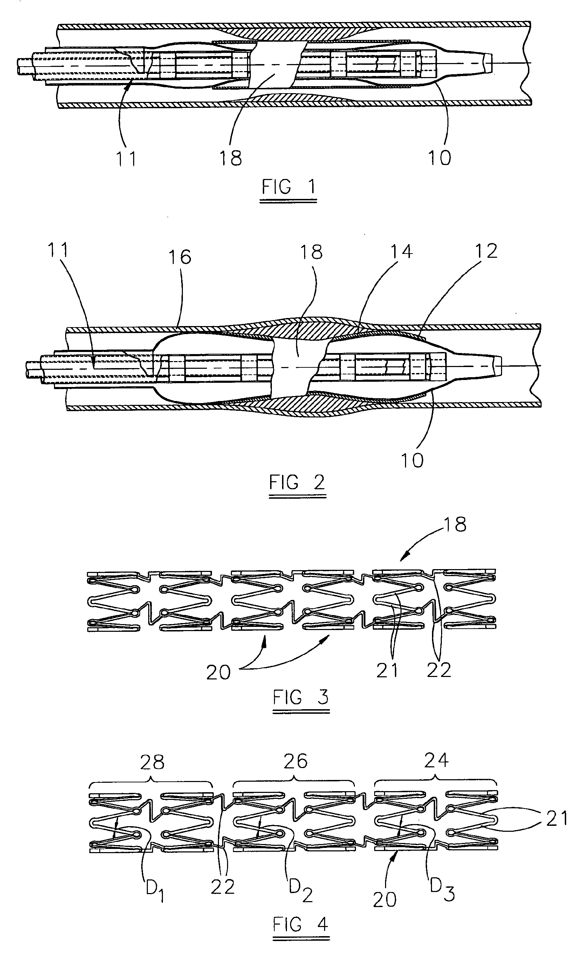

Referring to Figures 1 and 2 there is shown part of a stent implantation arrangement which comprises a balloon 10 carried by a suitable catheter arrangement 11 to permit inflation and deflation thereof. Figure 1 illustrates the stent implantation arrangement with the balloon 10 in a deflated state, Figure 2 illustrating the arrangement with the balloon 10 in an inflated state. As shown in Figure 2, the balloon 10 is designed in such a manner that, when inflated, it is not of generally cylindrical form, but rather is of continuously varying diameter and includes a useful length having a first part or region 12 of relatively large diameter, a second part or region 14 of reduced diameter, and a third part or region 16 of larger diameter. By way of example, the first region may be of diameter 7mm, the second region 6mm and the third region 8mm. The balloon is manufactured in such a manner that the balloon material has a "memory" so as to always adopt the desired shape when inflated to a given pressure. During manufacture, the balloon material is heated whilst it is being inflated to the desired shape within a mould of that shape, the mould typically being of copper construction. After the material has cooled, the balloon is deflated. Upon reinflation, the balloon will always assume the desired shape when inflated to a given pressure. A number of materials can be used in the manufacture of these balloons, including PET and Nylon. The walls of such a balloon are of good flexibility thus, when deflated, the balloon folds to a very small diameter. It will be appreciated that this manufacturing technique can be used to produce balloons of a wide variety of shapes, for example conical, tapered, dog-bone shaped, or of stepped diameter.

Figure 1 illustrates the balloon 10 in the deflated form prior to use in implanting a stent within a blood vessel of a patient. As illustrated in Figure 1, a stent 18 is crimped over the deflated balloon 10, the stent extending over the useful length of the balloon 10 including the parts 12, 14, 16 of the balloon 10 which will, when the balloon 10 is inflated, be of differing diameters. The stent 18 may take a wide range of forms, and so little detail of the stent is shown in Figures 1 and 2. Further details of suitable stent designs are set out hereinafter.

In use, the balloon 10 with the stent 18 crimped thereon is inserted into a blood vessel of a patient within which the stent 18 is to be implanted. The balloon 10 and stent 18 are manipulated using the catheter arrangement 11 to manoeuvre the balloon 10 and stent 18 to the position in which the stent 18 is to be implanted. Once this position has been reached, the balloon 10 is inflated.

As illustrated in Figure 2, which shows the arrangement with the balloon 10 in its inflated condition, as the stent 18 extends over the various regions 12, 14, 16 of the balloon 10 of different diameter, once the balloon 10 has been inflated to its fully expanded condition, the balloon 10 and stent 18 will not be of generally cylindrical form, but rather the balloon 10 will adopt the "memorised" shape and the stent 18 will be shaped to conform, generally, with the shape of the part of the balloon 10 over which it lies.

After the balloon 10 has been inflated to expand the stent 18 to the condition shown in Figure 2, as described above, a vacuum is applied to cause the balloon 10 to collapse to its deflated form. Although the balloon 10 collapses to this form, the stent 18 remains in the expanded condition. It will thus be appreciated that by appropriate manipulation of the catheter arrangement 11 of which the balloon 10 forms part, the balloon 10 can be withdrawn leaving the stent 18 implanted within the patient.

The invention is suitable for use with a wide range of stent designs. It will be appreciated, however, that certain stent designs have advantages over other designs of stent. For example, in order to ensure that the stent 18 remains positioned over the parts of the balloon 10 of various diameters, it is desirable to use a stent 18 designed in such a manner that the length thereof reduces by only a small amount as the stent 18 is expanded from its reduced diameter condition as shown in Figure 1 to its expanded condition shown in Figure 2. Further, in order to accommodate the changes in diameter along the length of the stent 18, it will be appreciated that the stent 18 is conveniently of relatively flexible malleable form.

One particularly suitable design of stent is illustrated in Figure 3. The stent illustrated in Figure 3 comprises a length of a surgical grade stainless steel of tubular form into which a series of openings have been cut, for example by laser cutting, resulting in the stent essentially comprising a set of corrugated rings 20, each ring 20 being composed of a series of interconnected struts 21, some of the corrugations of each ring 20 being connected to some of the corrugations of an adjacent one of the rings 20 through a series of linkages 22 each of which is of generally 'S' shape. It will be appreciated that the use of the linkages 22 to inter-connect the various corrugated rings 20 allows each ring 20 to articulate relative to the adjacent rings 20 giving rise to a large degree of malleability and flexibility. The corrugations of each ring 20 allow each ring 20 to be expanded from the small diameter condition illustrated in Figure 3 to a condition in which each ring 20 is of enlarged diameter, such expansion resulting in the corrugations of each ring 20 becoming spaced by a greater degree.

Although a stent of this type is particularly suitable for use with the implantation arrangement of the present invention, as it is of very good malleability, flexibility, in a more preferable arrangement the stent 18 is designed such that its resistance to radial expansion is not uniform along the length of the stent 18, but rather varies to conform with the shape of the balloon 10. The radial resistance to expansion is conveniently relatively low for those parts of the stent 18 which are to be expanded to a large diameter, the resistance to expansion being higher for those regions of the stent 18 which are to be expanded to a smaller diameter. Thus, if such a stent is to be used in the stent implantation arrangement of Figures 1 and 2, the stent 18 should include a first region arranged to overlay the first region 12 of the balloon 10, a second region arranged to overlay the second region 14 of the balloon 10 and a third region arranged to overlay the third region 16 of the balloon 10. The third region of the stent 18 should have a relatively low resistance to radial expansion, the second region of the stent having a relatively high resistance to radial expansion, the first region of the stent 18 having an intermediate resistance to radial expansion.

Figure 4 illustrates a stent similar to that of Figure 3 but including first, second and third regions 24, 26, 28 arranged to overlie the first, second and third regions 12, 14, 16 of the balloon 10 shown in Figure 2, the first region 24 having a strut thickness or width D₃ for example, of 0.18mm, the second region 26 having a strut thickness or width D₂, for example, of 0.19mm and the third region 28 having a strut thickness or width D₁, for example of 0.17mm. By arranging the first, second and third regions 24, 26, 28 of the stent 18 to overlie the first, second and third regions 12, 14, 16 of the balloon 10, it will be appreciated that the radial resistance to expansion of the stent works with the balloon 10 in ensuring that the stent 18 is expanded to the desired shape when the balloon 10 is inflated.

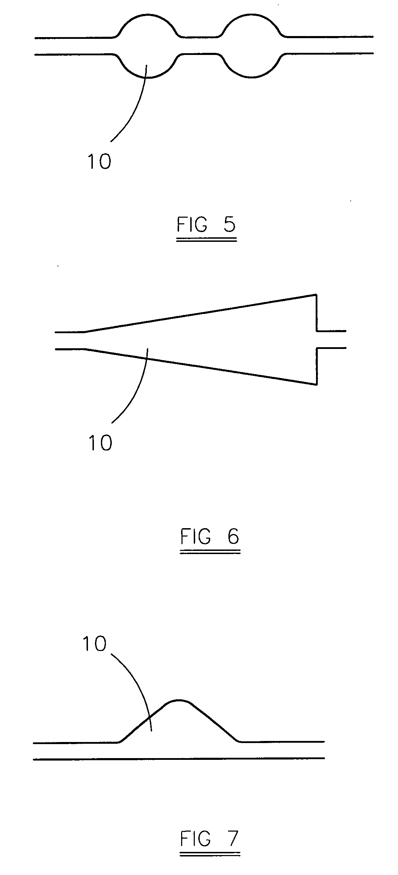

Although in the description hereinbefore, the balloon 10 is shaped to take a dog bone shape when expanded, so that the stent 18, when implanted, includes regions of three different diameters, it will be appreciated that this need not be the case. The shape of the balloon 10 can be chosen to produce a stent which, when implanted, takes a shape to suit the geometry of the part of the vessel within which the stent is to be implanted. The choice of the shape will depend upon the geometry of the vessel and upon the position of any obstructions or partial obstructions within the blood vessel. By way of example only, the balloon could be shaped to include two enlarged diameter regions of the same diameter separated by a small diameter region (see Figure 5), an additional region of large diameter, or could be of tapering diameter (see Figure 6). The balloon could, additionally, be arranged to be eccentric to the vessel or passage (see Figure 7). It will be appreciated, however, that the invention is not restricted to this range of shapes, and that the invention covers any implantation arrangement in which a balloon which, when inflated, assumes a non-cylindrical shape is used to implant a stent so that the implanted stent is also not of cylindrical form as defined by the appended claims.

## Claims

1. A stent implantation arrangement comprising an inflatable balloon (10) and a stent (18) mounted upon the balloon (10) by being crimped over the balloon, the balloon (10) being of the type which, when inflated to a given pressure, has a useful length which adopts a non-cylindrical shape, and **characterised in that** the stent is crimped over the useful length of the balloon which, when inflated, adopts the non-cylindrical shape.

2. An arrangement as claimed in Claim 1, **characterised in that** the balloon (10), when inflated, includes a pair of relatively large diameter regions (12, 16) separated by a relatively small diameter region (14), the stent (18) overlying the parts of the balloon (10) which, when inflated, form both of the relatively large diameter regions (12, 16).

3. An arrangement as claimed in Claim 2, **characterised in that** the balloon (10) includes at least one further region of a different diameter.

4. An arrangement as claimed in any one of Claims 1 to 3, **characterised in that** the balloon (10) is of continuously varying diameter along its length.

5. An arrangement as claimed in any one of the preceding claims, **characterised in that** the balloon, when inflated, includes a relatively large diameter region (12) and a relatively small diameter region (14) the stent (18) having a relatively low radial resistance to expansion in a first part (24) thereof which overlays the part (12) of the balloon which, when inflated, is of relatively large diameter, and a higher radial resistance to expansion in a second part (26) thereof which overlays the part (14) of the balloon (10) which, when inflated, is of relatively small diameter.

6. An arrangement as claimed in Claim 5, **characterised in that** the stent comprises a plurality of radially expandable rings made up of interconnected struts, and wherein the first region (24) of the stent (18) has a different strut thickness to the second region (26) thereof.

7. An arrangement as claimed in Claim 5 or Claim 6, **characterised in that** the stent (18) comprises a plurality of corrugated rings (20) interconnected by 'S' shaped linkages (22).

8. A stent comprising a tubular body defined by a plurality of corrugated rings (20) interconnected by 'S' shaped linkages (22), each corrugated ring (20) comprising a plurality of struts interconnected with one another, and **characterised in that** the struts of at least one of the rings (20) are of a different thickness to the struts of at least one other of the rings (20).
